(19) Europäisches Patentamt
European Patent Office
Office européen des brevets

(11) Veröffentlichungsnummer: **0 253 303 B1**

(12) **EUROPÄISCHE PATENTSCHRIFT**

(45) Veröffentlichungstag der Patentschrift: **03.06.92**

(51) Int. Cl.[5]: **C07C 229/04**, C07K 3/20, B01J 20/32

(21) Anmeldenummer: **87109892.7**

(22) Anmeldetag: **08.07.87**

# (54) Neue Metallchelatharze.

(30) Priorität: **10.07.86 CH 2782/86**

(43) Veröffentlichungstag der Anmeldung:
**20.01.88 Patentblatt 88/03**

(45) Bekanntmachung des Hinweises auf die Patenterteilung:
**03.06.92 Patentblatt 92/23**

(84) Benannte Vertragsstaaten:
**AT BE CH DE FR GB IT LI LU NL SE**

(56) Entgegenhaltungen:

**JOURNAL OF APPLIED BIOCHEMISTRY, Band 4, 1982, Seiten 203-208, Academic Press, INC., New York, US; B. LÖNNERDAL et al.: "Metal chelate affinity chromatography of proteins"**

**TRENDS IN BIOTECHNOLOGY, Band 3, Nr. 1, Januar 1985, Seiten 1-7, Elsevier Science Publishers B.V., Amsterdam, NL; E. SULKOWSKI: "Purification of proteins by IMAC"**

**JOURNAL OF CHROMATOGRAPHY, Band 411, 18. Dezember 1987, Amsterdam, NL; E. HOCHULI et al.: "New metal chelate adsorbent selective for proteins and peptides containing neighbouring histidine residues"**

(73) Patentinhaber: **F. HOFFMANN-LA ROCHE AG**
**Postfach 3255**
**CH-4002 Basel(CH)**

(72) Erfinder: **Döbeli, Heinz, Dr.**
**Lupsingerstrasse 22**
**CH-4417 Ziefen(CH)**
Erfinder: **Hochuli, Erich, Dr.**
**Kirchackerstrasse 25**
**CH-4411 Arisdorf(CH)**

(74) Vertreter: **Lederer, Franz, Dr. et al**
**Patentanwalt Dr. Franz Lederer Lucile-Grahn-Strasse 22**
**W-8000 München 80(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft neue Harze, die für die Metallchelat-Chromatographie geeignet sind und deren Herstellung, sowie die Verwendung dieser Metallchelatharze zur Reinigung von Proteinen, insbesondere solchen, die benachbarte Histidinreste enthalten.

1975 wurde von Porath et al. [Nature 258, 598-599 (1975)] die Metallchelat-Affinitäts-Chromatographie, eine neue Reinigungsmethode für Proteine, eingeführt. Diese neue Technik wurde inzwischen vielerorts erfolgreich angewendet und bereits in Uebersichtsartikeln abgehandelt [Lönnerdal, B. and Keen C.L., J. Appl. Biochem. 4, 203-208 (1982); Sulkowski, E., Trends in Biotechnology 3, 1-7 (1985)]. Die Metallchelat-Affinitäts-Chromatographie basiert auf der Erkenntnis, dass durch Chelatbindung an ein Chromatographiegel gebundene (immobilisierte) Metallionen wie $Cu^{2+}$ und $Zn^{2+}$ mit an der Oberfläche von Proteinen befindlichen Elektronendonorgruppen, insbesondere der Imidazol-Seitenkette des Histidins, in reversible Wechselwirkung treten können. Bei einem pH-Wert, bei dem die Elektronendonorgruppe mindestens teilweise in nicht-protonierter Form vorliegt wird das Protein an das Chromatographiegel (z.B. Agarose) gebunden und kann anschliessend, beispielsweise mittels eines Puffers mit niedrigem pH-Wert, bei dem die Elektronendonorgruppe protoniert ist, eluiert werden. Als Chelatbildner hat sich beispielsweise Iminodiessigsäure sehr bewährt, die über einen sogenannten Spacer an die Trägermatrix des Harzes gebunden wird.

Ein ideales Chelat-Harz für die Reinigung von Biopolymeren muss also einerseits die Metallionen stark komplexieren und andererseits reversible Wechselwirkungen zwischen Metallionen und Proteinen erlauben. Immobilisierte Iminodiessigsäure erfüllt diese Bedingungen für $Cu^{II}$-Ionen weitgehend, für $Ni^{II}$-Ionen nur begrenzt, da letztere nur schwach gebunden und schon beim Beladen mit dem Proteingemisch oft ausgewaschen werden. Andererseits sind $Ni^{II}$-Chelatharze für die Reinigung von biologischem Material von besonderem Interesse, weil $Ni^{2+}$ eine höhere Koordinationszahl besitzt: $Ni^{II}$-Ionen können sechs Liganden komplexieren, $Cu^{II}$-Ionen vorzugsweise vier. In Nickelkomplexen stehen für die Verankerung der Metallionen im Harz vier Valenzen und für die Wechselwirkungen zwischen Metallionen und Biopolymeren zwei Valenzen zur Verfügung.

Es hat bisher nicht an Versuchen gefehlt Chelatharze mit möglichst grosser Affinität zu einem Metallion herzustellen. Als komplexbildende Komponente wurden z.B. N,N,N'-Ethylen-diamintriessigsäure [Haner, M. et al., Anal. Biochem. 138, 229-234 (1984)] und 1,3-Diaminopropan N,N,N',N'-tetraessigsäure [Moyers, E.M. and J.S. Fritz, Anal. Chem. 49, 418-423 (1977)] eingesetzt. Diese Harze haben jedoch den Nachteil, dass die Wechselwirkungen zwischen Metallionen und Biopolymeren nicht optimal sind.

Nitrilotriessigsäure ist ein vierzähniger Chelatbildner. Immobilisierte Nitrilotriessigsäure wäre ein geeignetes Chelatharz für Metallionen mit der Koordinationszahl sechs, da zwei Valenzen für die reversible Bindung des Biopolymeren zur Verfügung stehen. Ein solches Metallchelatharz sollte sich besonders für die Bindung von Proteinen mit zwei benachbarten Histidinen auf seiner Oberfläche eignen.

Nitrilotriessigsäure kann aber nicht analog zu Imminodiessigsäure an einen Träger gebunden werden, ohne dass ihre Fähigkeit zur Chelatbindung sich wesentlich verschlechtert. Dieses Problem konnte durch die Herstellung neuer Nitrilotriessigsäurederivate der Formel

$$NH_2\text{-}(CH_2)_x\text{-}CH(COOH)\text{-}N(CH_2COOH)_2 \qquad I$$

worin x 2, 3 oder 4 bedeutet,
und deren Immobilisierung an eine Trägermatrix via einen Spacer gelöst werden.

Die vorliegende Erfindung betrifft daher Nitrilotriessigsäurederivate der vorstehend genannten Formel und deren Salze sowie Verfahren zu deren Herstellung. Besonders bevorzugte erfindungsgemässe Nitrilotriessigsäurederivate sind N-[3-Amino-1-carboxypropyl]-iminodiessigsäure und N-[5-Amino-1-carboxypentyl]-iminodiessigsäure.

Die vorliegende Erfindung betrifft ferner Metallchelatharze, die sich auf Grund ihrer Metallchelatgruppen für die Reinigung von Proteinen, insbesondere solchen, die benachbarte Histidine enthalten, eignen, sowie Verfahren zu deren Herstellung.

Die erfindungsgemässen Metallchelatharze sind durch die allgemeinen Formel Trägermatrix-Spacer-$NH\text{-}(CH_2)_x\text{-}CH(COOH)\text{-}N(CH_2COO^-)_2 \quad Ni^{2+}$ definiert, worin x 2, 3 oder 4 bedeutet.

Als Trägermatrix kommen Materialien in Frage, wie sie in der Affinitäts- und Gelchromatographie verwendet werden, beispielsweise vernetzte Dextrane, Agarose (insbesondere in der unter dem Warenzeichen Sepharose® bekannten Form) oder Polyacrylamide.

Als Spacer kommen die aus der Affinitäts-Chromatographie bereits bekannten Spacer-Gruppen in Frage, wobei die Gruppen $\text{-O-}CH_2\text{-}CH(OH)\text{-}CH_2\text{-}$ und -O-CO- bevorzugt sind .

Besonders bevorzugte erfindungsgemässe Chelatharze sind solche der Formeln

[Agarose- bzw. Sepharose®CL 6B]-$O\text{-}CH_2\text{-}CH(OH)\text{-}CH_2\text{-}NH\text{-}(CH_2)_4\text{-}CH(COOH)\text{-}\text{-}N(CH_2COO^-)_2\ Ni^{2+}$

und

Agarose $-O-CO-NH-(CH_2)_2-CH(COOH)-N-(CH_2COO^-)_2\ Ni^{2+}$.

Die Herstellung der erfindungsgemässen Nitrilotriessigsäurederivate kann in an sich bekannter Weise, durch Umsetzung einer N-terminal geschützten Verbindung der Formel $R-HN-(CH_2)_x-CH-(NH_2)-COOH$, worin R eine Aminoschutzgruppe und x 2, 3 oder 4 bedeuten, mit Bromessigsäure in alkalischem Medium und anschliessender Abspaltung der Schutzgruppe erfolgen. Eine bevorzugte Aminoschutzgruppe ist der Benzyloxycarbonylrest (Z), der durch katalytische Hydrierung, vorzugsweise mit Pd/C, wieder entfernt werden kann. Auf diese Weise können $N^{\gamma}$-Z-L-2,4-Diaminobuttersäure und $N^{\epsilon}$-Z-L-Lysin in die vorstehend genannten besonders bevorzugten Nitrilotriessigsäurederivate überführt werden.

Die Herstellung der erfindungsgemässen Chelatharze kann in an sich bekannter Weise erfolgen, wobei zunächst die Trägermatrix funktionalisiert wird (Einführung des Spacers) und dann das gewünschte Nitrilotriessigsäurederivat kovalent an den Spacer gebunden wird.

Bei Agarose als Trägermatrix setzt man beispielsweise mit Epibromhydrin in alkalischem Medium um, so dass man Oxiran-agarose erhält, die

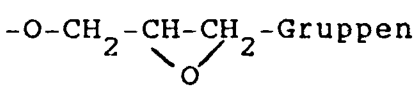

enthält. Die Oxiran-agarose kann dann in an sich bekannter Weise durch Umsetzung mit einem erfindungsgemässen Nitriloessigsäurederivat, vorzugsweise mit N-[3-Amino-1-carboxypropyl]-iminodiessigsäure oder N-[5-Amino-1-carboxypentyl]-iminodiessigsäure, in alkalischem Medium und anschliessendem Waschen mit einer Nickelsalzlösung, beispielsweise mit Nickelsulfat, in das gewünschte erfindungsgemässe Chelatharz übergeführt werden. Ist in speziellen Fällen der Einsatz eines anderen Metallions (z.B. Co, Cd) vorteilhaft, so kann das entsprechende Metallchelat leicht durch Umsetzung des Harzes mit einem geeigneten Metallsalz erhalten werden. Statt Epibromhydrin kann auch Epichlorhydrin eingesetzt werden. Als Agarose verwendet man zweckmässigerweise ein standardisiertes Produkt, vorzugsweise Sepharose® der Firma Pharmacia, Uppsala, Schweden. Besonders geeignet ist Sepharose® CL-6B. In analoger Weise können Polyacrylharze, die freie Hydroxygruppen enthalten, wie vorstehend angegeben, in erfindungsgemässe Chelatharze überführt werden. Bei Verwendung von Kationenaustauscherharzen als Matrix kann die Kopplung des Nitrilotriessigsäurederivats direkt unter Ausbildung einer Amidbindung erfolgen.

Zur Herstellung der erfindungsgemässen Chelatharze können auch im Handel erhältliche, bereits funktionalisierte Trägermatrizes verwendet werden. Eine besonders bevorzugte funktionalisierte Trägermatrix im Zusammenhang mit der vorliegenden Erfindung ist Imidazolcarbamat-agarose, die

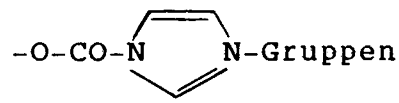

enthält, und die unter dem Markennamen Reacti-gel™ der Firma Pierce, Rockford, IL, USA, im Handel ist.

Es hat sich gezeigt, dass sich die erfindungsgemässen Chelatharze durch eine besonders hohe Spezifität gegenüber Peptiden und Proteinen, die benachbarte Histidinreste enthalten, auszeichnen und daher besonders geeignet sind für die Reinigung von Proteinen mit benachbarten Histidinresten, insbesondere solchen, die 2 benachbarte Histidinreste enthalten. Der Ausdruck "benachbarte Histidinreste" bezieht sich auf die Anordnung der Histidinreste der betreffenden Peptide und Proteine im dreidimensionalen Raum, d.h. an der Oberfläche der Verbindungen. Die Nachbarschaft der Histidinreste kann bereits aufgrund der Primärstruktur gegeben sein oder erst durch die Sekundär- und/oder Tertiärstruktur zustandekommen. Die erfindungsgemässen Chelatharze eignen sich daher zur Reinigung von nativen und denaturierten Proteinen, die mehrere, insbesondere benachbarte, vorzugsweise unmittelbare benachbarte Histidinreste enthalten.

Die erfindungsgemässen Chelatharze können batch-weise oder in kontinuierlich zu betreibenden Säulen verwendet werden. Die erfindungsgemässen Chelatharze werden vor der Beladung mit Protein zweckmässigerweise mit einem wässrigen Puffer, der selber keine Chelate mit Nickel bildet, vorzugsweise einem Phosphatpuffer, pH 8, äquilibriert. Der Aequilibrierungspuffer (wie auch die Elutionspuffer) kann ein Denaturierungsmittel bzw. ein Detergenz, beispielsweise Guanidin•HCl, Harnstoff oder Triton enthalten. Der Zusatz eines solchen Denaturierungsmittels oder Detergenzes ermöglicht problemloses Arbeiten selbst mit Proteinen, die in wässriger Lösung extrem schwer löslich sind, wie beispielsweise Membranproteine. Die Elution des Proteins kann bei konstantem pH-Wert oder mit linear oder diskontinuierlich fallenden pH-Gradienten durchgeführt werden. Die optimalen Elutionsbedingungen hängen von der Menge und Art der vorhandenen Verunreinigungen, der Menge des zu reinigenden Materials, den Säulendimensionen

usw. ab und sind zweckmässigerweise von Fall zu Fall zu bestimmen.

Die folgenden Beispiele illustrieren die Herstellung erfindungsgemässer Nitrilotriessigsäurederivate sowie die Herstellung erfindungsgemässer Metallchelatharze und deren Verwendung bei der Reinigung von Proteinen mit benachbarten Histidinresten.

Beispiel 1

41,7 g Bromessigsäure wurden in 150 ml 2N Natronlauge gelöst und auf 0 °C gekühlt. Dazu wurde unter Rühren eine Lösung von 42 g $N^{\epsilon}$-Z-L-Lysin in 225 ml 2N Natronlauge bei 0 °C langsam zugetropft. Nach 2 Stunden wurde die Kühlung abgestellt und über Nacht weitergerührt. Dann wurde das Reaktionsgemisch während 2 Stunden bei 50 °C gehalten und anschliessend wurden 450 ml 1N Salzsäure zugesetzt. Nachdem das Gemisch abgekühlt war wurden die ausgeschiedenen Kristalle abfiltriert. Das Produkt wurde in 1N Natronlauge gelöst und mit der gleichen Menge 1N Salzsäure erneut gefällt und abfiltriert. Es wurden 40 g N-[5-Benzyloxycarbonylamino-1-carboxypentyl]-iminodiessigsäure in Form weisser Kristalle, Smp. 172-174 °C (Zers.), $[\alpha]_D$ = +9,9° (c = 1; 0,1 N NaOH), erhalten.

7,9 g des erhaltenen Lysinderivats wurden in 49 ml 1N Natronlauge gelöst und nach Zusatz einer Spatelspitze 5% Pd/C bei Raumtemperatur und Normaldruck hydriert. Der Katalysator wurde abfiltriert und das Filtrat eingedampft. Es resultierten 6,2 g N-[5-Amino-1-carboxypentyl]-iminodiessigsäure, deren Struktur, $NH_2$-$(CH_2)_4$-CH(COOH)-$N(CH_2COOH)_2$, durch das NMR-Spektrum bestätigt wurde.

100 ml Sepharose® CL-6B (Pharmacia) wurden auf einer Glasfilternutsche zweimal mit ca. 500 ml Wasser gewaschen und dann in einem 500 ml Rundkolben mit 16 ml 4N Natronlauge und 8,22 ml Epibromhydrin 4 Stunden bei 30 °C umgesetzt. Das Totalvolumen des Reaktionsgemisches war 200 ml. Anschliessend wurde die aktivierte Sepharose abfiltriert, mit Wasser neutral gewaschen und zurück in das Reaktionsgefäss transferiert. 6,5 g N-[5-Amino-1-carboxypentyl]-iminodiessigsäure wurden in 50 ml Wasser gelöst und zusammen mit 10,6 g festem Soda zur aktivierten Sepharose gegeben. Das Gemisch wurde bei 60 °C über Nacht langsam gerührt. Das resultierende Chelatharz mit der Formel [Sepharose®CL-6B]-O-$CH_2$-CH(OH)-$CH_2$-NK-$(CH_2)_4$-CH(COOH)-$N(CH_2COOH)_2$ (NTA-Harz) wurde anschliessend in einer Chromatographiesäule nacheinander mit 500 ml Wasser, 100 ml wässrigem $NiSO_4 \cdot 6H_2O$ (2 Gew.-%), 200 ml Wasser, 200 ml 0,2M Essigsäure (enthaltend 0,2M NaCl und 0,1 Gew./Vol.% Tween 20) und 200 ml Wasser gewaschen. Die Nickelionenkonzentration des resultierenden Chelatharzes der Formel [Sepharose®CL-6B]-O-$CH_2$-CH(OH)-$CH_2$-NH-$(CH_2)_4$-CH(COOH)-$N(CH_2COO^-)_2Ni^{2+}$ betrug etwa 7,1 Mikromol/ml.

Beispiel 2

Für einen qualitativen Vergleich der Stabilitäten der Nickelkomplexe von immobilisierter Iminodiessigsäure (IMA) und immobilisierter Nitrilotriessigsäure (NTA) wurden die beiden Nickelchelatharze mit einer wässerigen Lösung von Iminodiessigsäure eluiert und das Auswaschen der Nickelionen verfolgt.

In eine Chromtographiesäule (d = 1,6 cm) wurden 50 ml IMA-Harz der Formel Agarose-O-$CH_2$-CH(OH)-$CH_2$-$N(CH_2COOH)_2$ (Herstellung siehe Europäische Patentanmeldung Nr. 84101814.6, Veröffentlichungsnummer 118 808) gefüllt und gut mit Wasser gewaschen. Dann wurden bei einer Durchflussgeschwindigkeit von 100 ml/h 10 ml einer 0,012M $NiSO_4 \cdot 5H_2O$-Lösung in Wasser aufgetragen und anschliessend die Säule mit 70 ml Wasser gewaschen. Mit 0,1M wässriger Iminodiessigsäure (IMA), pH 7,0, wurde eluiert. Es wurden Fraktionen zu je 10 ml gesammelt. In den Fraktionen 10-19 konnten Nickelionen nachgewiesen werden (UV 390 nm).

In gleicher Weise wurden 50 ml NTA-Harz der Struktur [Sepharose®CL-6B]-O-$CH_2$-CH(OH)-$CH_2$-NH-$(CH_2)_4$-CH(COOH)-$N(CH_2COOH)_2$ in eine Chromatographiesäule (d = 1,6 cm) gefüllt, mit Wasser gewaschen, danach mit 10 ml 0,012M $NiSO_4 \cdot 5H_2O$ beladen, wieder mit Wasser gewaschen und mit 0,1M wässriger Iminodiessigsäure, pH 7,0, eluiert. Erst in den Fraktionen 30-34 konnten Nickelionen nachgewiesen werden (UV 390 nm), womit gezeigt ist, dass im neuen NTA-Harz die $Ni^{II}$-Ionen stärker gebunden sind als im bekannten IMA-Harz.

Beispiel 3

6,5 g Bromessigsäure wurden in 8,1 ml 4N Natronlauge gelöst und auf 0 °C gekühlt. Dazu wurde unter Rühren eine Lösung von 4,1 g $N^{\gamma}$-Benzyloxycarbonyl-L-2,4-diaminobuttersäure in 24,4 ml 2N Natronlauge zugetropft. Nach 2 Stunden wurde die Kühlung abgestellt und über Nacht weitergerührt. Dann wurde das Reaktionsgemisch während 2 Stunden bei 50 °C gehalten und anschliessend wurden 12,2 ml 4N Salzsäure zugesetzt. Nachdem das Gemisch abgekühlt war, wurden die ausgeschiedenen Kristalle abfiltriert. Das Produkt wurde in 2N Natronlauge gelöst und mit 6,1 ml 4N Salzsäure erneut gefällt und abfiltriert. Es wurden 5 g N-[3-Benzyloxycarbonylamino-1-carboxypropyl] -iminodiessigsäure in Form weisser Kristalle, Smp. 136-138 ° C (Zers.), erhalten.

2,9 g des erhaltenen Buttersäurederivates wurden in 16 ml 1N Natronlauge gelöst und nach Zusatz einer Spatelspitze 5% Pd/C bei Raumtemperatur und Normaldruck hydriert. Der Katalysator wurde abfiltriert und das Filtrat eingedampft. Es resultierten 2,2 g N-[3-Amino-1-carboxypropyl]-iminodiessigsäure, deren Struktur, $NH_2$-$(CH_2)_2$-CH-(COOH)-N$(CH_2COOH)_2$, durch das NMR-Spektrum bestätigt wurde.

Eine Lösung von 1,9 g der erhaltenen N-[3-Amino-1-carboxypropyl]-iminodiessigsäure in 50 ml Wasser wurde mit 2,6 g fester Soda versetzt, zu dem auf 0 ° C gekühlten Gemisch, wurden 50 ml mit Imidazolcarbamat aktivierte Agarose (Reacti-Gel™ der Firma Pierce) zugegeben. Nach 15 Stunden Inkubation bei 0 ° C wurde das resultierende Chelatharz der Formel Agarose-O-CO-NH-$(CH_2)_2$-CH(COOH)-N$(CH_2COOH)_2$ abfiltriert, mit Wasser gewaschen und wie in Beispiel 1 beschrieben mit $Ni^{II}$-Ionen beladen. Die Nickelionenkonzentration des resultierenden Chelatharzes der Formel Agarose-O-CO-NH-$(CH_2)_2$-CH(COOH)-N-$(CH_2COO^-)Ni^{2+}$ betrug 3,1 Mikromol/ml.

## Beispiel 4

Eine Säule (Ø 1 cm, Länge = 4,8 cm) wurde mit metallfreiem Chelatharz der Formel [Sepharose®CL-6B]-O-$CH_2$-CH(OH)-$CH_2$-NH-$(CH_2)_4$-CH(COOH)-N$(CH_2COOH)_2$ (NTA-Harz) gefüllt und das Harz durch Spülen mit dem dreifachen Säulenvolumen 0,1M $NiSO_4 \cdot 5H_2O$ und anschliessendes Waschen mit dem dreifachen Säulenvolumen 0,2M Essigsäure in die Nickelform gebracht. Anschliessend wurde mit 0,1M Natriumphosphatpuffer (pH 8,0 und 0,5M NaCl äquilibriert (Durchfluss jeweils 13,2 ml/Std.).

1 mg eines Modellpeptids der Formel His-His-Leu-Gly-Gly-Ala-Lys-Glu-Ala-Gly-Asp-Val wurde in 1 ml Aequilibrierpuffer aufgenommen und auf die Säule aufgetragen. Durch Waschen mit 0,1M Imidazol in 0,1M Natriumphosphat, pH 8,0 und 0,5M NaCl konnte das Modellpeptid eluiert werden. Der Nachweis im Eluat erfolgte mit Ninhydrin nach Moore, S. und Stein, W. [J. Biol. Chem. 176, 367-388 (1948)].

## Beispiel 5

In zu Beispiel 4 analoger Weise wurde eine Säule (Ø = 1 cm, Länge = 4,8 cm) mit NTA-Harz gefüllt und das Harz in die Nickelform gebracht. Nach dem Waschen mit 0,2M Essigsäure wurde die Säule mit 7M Guanidin•HCl in 0,1M Natriumphosphatpuffer (pH 8,0) äquilibriert.

Verschiedene Mengen (bis zu 12,7 mg) eines Modellpeptids mit der Formel Asp-Arg-Val-Tyr-Ile-His-Pro-Phe-His-Leu-Val-Ile-His-Ser wurden in 1 ml 7M Guanidin•HCl und 0,1M Natriumphosphat (pH 8,0) gelöst und auf die Säule aufgetragen. Dieses Peptid ist in 7M Guanidin•HCl sehr gut, jedoch in 0,1M Natriumphosphat und 0,5M NaCl schlecht löslich. Die Elution erfolgte durch stufenweises Senken des pH-Wertes. Das Peptid wurde mittels UV-Spektrometrie bei $\lambda$ = 280 nm nachgewiesen.

Als Vergleichssubstanzen wurden Trypsin aus Rinderpankreas und Cytochrom C aus Pferdeherz verwendet. Keines der beiden Proteine bindet bei pH 8 an das NTA-Harz. Offensichtlich spielt die Anordnung der Histidine eine ausschlaggebende Rolle. Beim Trypsin befinden sich drei Histidine in den Positionen 29, 46 und 79, die trotz Entfaltung der Struktur durch 7M Guanidin nicht in der Lage sind, einen stabilen Komplex zu bilden und beim Cytochem C sind die beiden Histidine zwar räumlich benachbart (Positionen 18 und 26), aber nicht in der Lage, einen zweizähnigen Liganden zu bilden, da ein Histidin an das Häm-Eisen gebunden ist.

## Beispiel 6

Laktatdehydrogenase-Isoenzyme sind tetramere Proteine mit einem Molekulargewicht von 140'000. Die Isoenzyme aus Schwein sind weitgehend homolog mit Ausnahme der aminoterminalen Region. Diese befindet sich an der Proteinoberfläche. Das Herztyp-Isoenzym besitzt in dieser Region kein Histidin, das Muskeltyp-Isoenzym hingegen drei, darunter die Sequenz His-Val-Pro-His [L.Li et al., J. Biol. Chem. 258, 7029-7032 (1983)].

Wie in Beispiel 4 beschrieben wurde eine Säule (Ø = 1 cm, Länge = 4,8 cm) mit NTA-Harz gefüllt, das Harz in die Nickelform gebracht und mit 0,1M Natriumphosphatpuffer (pH 7,5) und 0,5M NaCl äquilibriert. 2 mg Laktatdehydrogenase aus Schweineherz ($H_4$-LOH) oder Schweinemuskel ($M_4$-LOH) wurden in 1,5 ml Aequilibrierpuffer aufgenommen und auf die Säule aufgetragen. Während $H_4$-LOH trotz ihrer 28 Histidinreste nicht adsorbiert wurde, wurde $M_4$-LOH bei pH 7,5 absorbiert und konnte durch senken des pH-Wertes auf 6 eluiert werden.

Dieses Experiment zeigt, dass das NTA-Harz äusserst selektiv ist für Proteine, die als Strukturelement benachbarte Histidine auf der Proteinoberfläche besitzen.

## Patentansprüche

**1.** Metallchelatharz der Formel

Trägermatrix-Spacer-NH-$(CH_2)_x$-CH(COOH)-N-$(CH_2COO^-)_2$ $Ni^{2+}$ (I)

worin X 2, 3 oder 4 bedeutet.

**2.** Metallchelatharz gemäss Anspruch 1, worin die Trägermatrix Agarose ist.

**3.** Metallchelatharz gemäss Anspruch 1, worin die Trägermatrix Sepharose®CL-6B ist.

**4.** Metallchelatharz gemäss einem der Ansprüche 1-3, worin der Spacer -O-CO- oder $-O-CH_2-CH(OH)-CH_2-$ ist.

**5.** Eine Verbindung der Formel

$H_2N-(CH_2)_x-CH(COOH)-N(CH_2COOH)_2$

worin x 2, 3 oder 4 bedeutet,
und deren Salze.

**6.** N-[3-Amino-1-carboxypropyl]-iminodiessigsäure.

**7.** N-[5-Amino-1-carboxypentyl]-iminodiessigsäure.

**8.** Verfahren zur Herstellung eines Metallchelatharzes gemäss einem der Ansprüche 1-4, dadurch gekennzeichnet, dass man eine Verbindung gemäss einem der Ansprüche 5-7 mit einer durch Spacergruppen funktionalisierten Trägermatrix umsetzt und anschliessend mit einer Nickelsalzlösung wäscht.

**9.** Verfahren gemäss Anspruch 8, dadurch gekennzeichnet, dass die funktionalisierte Trägermatrix Oxiran-Agarose ist, die

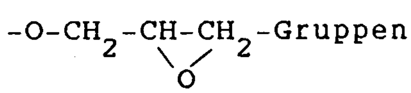

enthält.

**10.** Verfahren gemäss Anspruch 8, dadurch gekennzeichnet, dass die funktionalisierte Trägermatrix, Imidazolcarbamat-Agarose ist, die

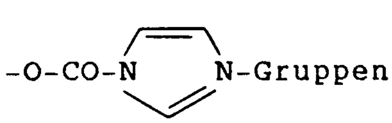

enthält.

**11.** Verfahren zur Herstellung einer Verbindung der Formel

$H_2N-(CH_2)_x-CH(COOH)-N(CH_2COOH)_2$

worin x 2, 3 oder 4 bedeutet,
dadurch gekennzeichnet, dass man eine N-terminal geschützte Verbindung der Formel

$R-HN-(CH_2)_x-CH(NH_2)-COOH$

worin R eine Aminoschutzgruppe und x 2, 3 oder 4 bedeuten,
mit mindestens 2 Aequivalenten Bromessigsäure umsetzt und aus dem resultierenden Reaktionsprodukt die Schutzgruppe abspaltet.

**12.** Verfahren gemäss Anspruch 11, dadurch gekennzeichnet, dass die Schutzgruppe Benzyloxycarbonyl (Z) ist und deren Abspaltung durch Hydrierung in Gegenwart von Pd/C erfolgt.

**13.** Verfahren zur Herstellung von [3-Amino-1-carboxypropyl]-iminodiessigsäure, dadurch gekennzeichnet, dass man $N^{\gamma}$-Benzyloxycarbonyl-L-2,4-diaminobuttersäure mit Bromessigsäure umsetzt und das resultierende Reaktionsprodukt in Gegenwart von Pd/C hydriert.

**14.** Verfahren zur Herstellung von N-[5-Amino-1-carboxypentyl]-iminodiessigsäure, dadurch gekennzeichnet, dass man $N^{\epsilon}$-Benzyloxycarbonyl-L-lysin mit Bromessigsäure umsetzt und das resultierende Reaktionsprodukt in Gegenwart von Pd/C hydriert.

**15.** Verfahren zur Reinigung von Proteinen, die mehrere benachbarte Histidinreste enthalten, dadurch gekennzeichnet, dass man sie der Affinitäts-Chromatographie an einem Metallchelatharz gemäss den Ansprüchen 1-4 unterwirft.

**16.** Verwendung eines Chelatharzes gemäss einem der Ansprüche 1-4 in der Metallchelat-Chromatographie.

**17.** Verwendung eines Chelatharzes gemäss einem der Ansprüche 1-4 zur Reinigung von Proteinen, die mehrere Histidinreste enthalten.

**18.** Verwendung eines Chelatharzes gemäss einem der Ansprüche 1-4 zur Reinigung von Proteinen, die 2 unmittelbar benachbarte Histidinreste enthalten.

## Claims

**1.** A metal chelate resin of the formula

Carrier matrix-spacer-NH-$(CH_2)_x$-CH(COOH)-N-$(CH_2COO^-)_2$ $Ni^{2+}$ (I)

wherein X signifies 2, 3 or 4.

2. A metal chelate resin according to claim 1, wherein the carrier matrix is agarose.

3. A metal chelate resin according to claim 1, wherein the carrier matrix is Sepharose®CL-6B.

4. A metal chelate resin according to any one of claims 1-3, wherein the spacer is -O-CO- or -O-$CH_2$-CH(OH)-$CH_2$-.

5. A compound of the formula

$H_2N$-$(CH_2)_x$-CH(COOH)-N$(CH_2COOH)_2$

wherein x signifies 2, 3 or 4,
and its salts.

6. N-[3-Amino-1-carboxypropyl]-iminodiacetic acid.

7. N-[5-Amino-1-carboxypentyl]-iminodiacetic acid.

8. A process for the manufacture of a metal chelate resin according to any one of claims 1-4, which process comprises reacting a compound according to any one of claims 5-7 with a carrier matrix functionalized with spacer groups and subsequently washing with a nickel salt solution.

9. A process according to claim 8, wherein the functionalized carrier matrix is oxirane-agarose which contains

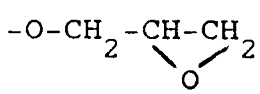

groups.

10. A process according to claim 8, wherein the functionalized carrier matrix is imidazolecarbamate-agarose which contains

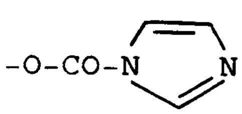

groups.

11. A process for the manufacture of a compound of the formula

$H_2N$-$(CH_2)_x$-CH(COOH)-N$(CH_2COOH)_2$

wherein x signifies 2, 3 or 4,
which process comprises reacting a N-terminal protected compound of the formula

R-HN-$(CH_2)_x$-CH($NH_2$)-COOH

wherein R signifies an amino protecting group and x signifies 2, 3 or 4,
with at least 2 equivalents of bromoacetic acid and cleaving off the protecting group from the resulting reaction product.

12. A process according to claim 11, wherein the protecting group is benzyloxycarbonyl (Z) and its cleavage is effected by hydrogenation in the presence of Pd/C.

13. A process for the manufacture of [3-amino-1-carboxypropyl]-iminodiacetic acid, which process comprises reacting $N^{\gamma}$-benzyloxycarbonyl-L-2,4-diaminobutyric acid with bromoacetic acid and hydrogenating the resulting reaction product in the presence of Pd/C.

14. A process for the manufacture of N-[5-amino-1-carboxypentyl]-iminodiacetic acid, which process comprises reacting $N^{\epsilon}$-benzyloxycarbonyl-L-lysine with bromoacetic acid and hydrogenating the resulting reaction product in the presence of Pd/C.

15. A process for the purification of proteins which contain several neighbouring histidine residues, which process comprises subjecting said proteins to affinity chromatography on a metal chelate resin as set forth in claims 1-4.

16. The use of a chelate resin according to any one of claims 1-4 in metal chelate chromatography.

17. The use of a chelate resin according to any one of claims 1-4 for the purification of proteins which contain several histidine residues.

18. The use of a chelate resin according to any one of claims 1-4 for the purification of proteins which contain 2 immediately neighbouring histidine residues.

## Revendications

**1.** Résine chélatante de métaux de formule

Matrice Matière support d'écartement -NH-$(CH_2)_x$-CH(COOH)-$N(CH_2COO^-)_2Ni^{2+}$ (I)

dans laquelle X vaut 2, 3 ou 4.

**2.** Résine chélatante de métaux selon la revendication 1, dans laquelle la matrice support est l'agarose.

**3.** Résine chélatante de métaux selon la revendication 1, dans laquelle la matrice support est le Sepharose®CL-6B.

**4.** Résine chélatante de métaux selon l'une des revendications 1-3, dans laquelle la matière d'écartement est -O-CO- ou -O-$CH_2$-CH(OH)-$CH_2$- .

**5.** Composé de formule

$H_2N$-$(CH_2)_x$-CH(COOH)-$N(CH_2COOH)_2$

dans lequel X vaut 2, 3 ou 4, et ses sels.

**6.** Acide N-[3-amino-1-carboxypropyl]-iminodiacétique.

**7.** Acide N-[5-amino-1-carboxypentyl]-iminodiacétique.

**8.** Procédé de préparation d'une résine chélatante de métaux selon l'une des revendications 1-4, caractérisé en ce qu'on fait réagir un composé selon l'une des revendications 5-7 avec une matrice support fonctionnalisée par des groupes d'écartement puis en ce qu'on lave avec une solution de sel de nickel.

**9.** Procédé selon la revendication 8, caractérisé en ce que la matrice support fonctionnalisée est l'oxiraneagarose, qui contient des groupes

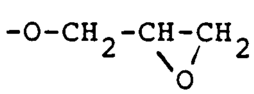

.

**10.** Procédé selon la revendication 8, caractérisé en ce que la matrice support fonctionnalisée est l'imidazolecarbamate-agarose qui contient des groupes

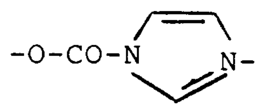

.

**11.** Procédé de préparation d'un composé de formule

$H_2N$-$(CH_2)_x$-CH(COOH)-$N(CH_2COOH)_2$

dans lequel x vaut 2, 3 ou 4, caractérisé en ce qu'on fait réagir un composé protégé N-terminal de formule

R-HN-$(CH_2)_x$-CH($NH_2$)-COOH

dans lequel R représente un groupe protecteur d'amino et x vaut 2, 3 ou 4, avec au moins 2 équivalents d'acide bromacétique, et on sépare le groupe protecteur du produit réactionnel résultant.

**12.** Procédé selon la revendication 11, caractérisé en ce que le groupe protecteur est le benzyloxycarbonyle (Z) et en ce que sa séparation s'effectue par hydrogénation en présence de Pd/C.

**13.** Procédé de préparation d'acide [3-amino-1-carboxypropyl]-iminodiacétique,caractérisé en ce qu'on fait réagir l'acide $N^{\gamma}$-benzyloxycarbonyl-L-2,4-diaminobutyrique avec de l'acide bromacétique et en ce qu'on hydrogène le produit réactionnel résultant en présence de Pd/C.

**14.** Procédé de préparation d'acide N-[5-amino-1-carboxypentyl]-iminodiacétique, caractérisé en ce qu'on fait réagir de la $N^{\epsilon}$-benzyloxycarbonyl-L-lysine avec de l'acide bromacétique et en ce qu'on hydrogène le produit réactionnel résultant en présence de Pd/C.

**15.** Procédé de purification de protéines qui contiennent plusieurs radicaux histidine voisins, caractérisé en ce qu'on les soumet à une chromatographie d'affinité sur une résine chélatante de métaux selon les revendications 1-4.

**16.** Application d'une résine chélatante selon l'une des revendications 1-4 dans la chromatographie des chélates de métaux.

**17.** Application d'une résine chélatante selon l'une des revendications 1-4 à la purification de protéines qui contiennent plusieurs radicaux histidine.

**18.** Application d'une résine chélatante selon l'une des revendications 1-4 à la purification de protéines qui contiennent 2 radicaux histidine immédiatement voisins.